(19) Europäisches Patentamt
European Patent Office
Office européen des brevets

(11) Numéro de publication: **0 073 301**
**B1**

# (12) FASCICULE DE BREVET EUROPEEN

(45) Date de publication du fascicule du brevet: **21.11.84**

(21) Numéro de dépôt: **82104703.2**

(22) Date de dépôt: **28.05.82**

(51) Int. Cl.³: **C 07 C 45/00**, C 07 C 49/203, C 07 C 49/217

(54) **Procédé pour la préparation de cétones alpha, bêta et bêta, gammainsaturées.**

(30) Priorité: **14.08.81 CH 5261/81**

(43) Date de publication de la demande: **09.03.83 Bulletin 83/10**

(45) Mention de la délivrance du brevet: **21.11.84 Bulletin 84/47**

(84) Etats contractants désignés: **CH DE FR GB LI**

(56) Documents cités: **FR - A - 2 170 238**

(73) Titulaire: **FIRMENICH SA, 1, route des Jeunes, CH-1211 Genève 8 (CH)**

(72) Inventeur: **Schulte-Elte, Karl-Heinrich, 44, chemin de Carabot, CH-1213 Onex (CH)**
Inventeur: **Snowden, Roger, 5, chemin des Palettes, CH-1212 Grand-Lancy (CH)**
Inventeur: **Müller, Bernard, 16, route de Malagnon, CH-1208 Genève (CH)**

(74) Mandataire: **Salvadori, Giuseppe, Dr., c/o Firmenich S.A. Case Postale 239, CH-1211 Genève 8 (CH)**

**EP 0 073 301 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention a trait à un procédé de fabrication de cétones insaturées, plus précisément $\alpha,\beta$- et $\beta,\gamma$-insaturées.

L'art antérieur fait état de nombreuses méthodes synthétiques pour la préparation des cétones insaturées, toutefois à ce jour, il ne semble pas exister une méthode pratique de transformation directe d'esters en cétones qui soit tout à la fois économique et techniquement intéressante.

La présente invention apporte donc une solution nouvelle à ce problème.

Le procédé de l'invention consiste à traiter au moyen d'une base forte, et dans un solvant organique inerte, un carbinol diallylique de formule

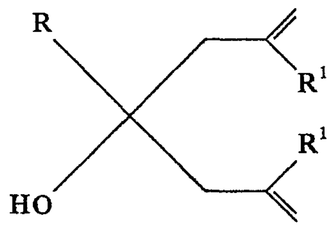

(I)

dans laquelle le symbole R représente un radical alkyle primaire, secondaire ou tertiaire ou un reste phényle, substitué ou non, et le symbole $R^1$ représente un atome d'hydrogène ou un radical alkyle, pour fournir une cétone de formule

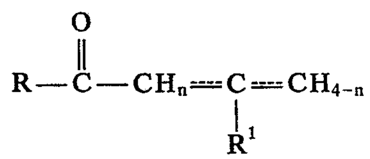

(II)

possédant une double liaison dans l'une des positions indiquées par les pointillés et dans laquelle les symboles R et $R^1$ ont le sens indiqué ci-dessus et l'indice n vaut 1 ou 2.

L'invention est définie par les revendications.

La méthode particulière de préparation suivie sera illustrée à l'aide des exemples qui suivent.

La réaction, qui caractérise le procédé de l'invention, consiste formellement en un clivage anionique, lequel est effectué par l'action d'une base forte. A cet effet, on peut utiliser des bases minérales ou organiques telles que les hydrures ou les alkoxydes d'un métal alcalin, le sodium et le potassium de préférence. Parmi lesdites bases, il convient de citer tout particulièrement l'hydrure de sodium ou potassium, le tert-butylate de sodium ou potassium, le tert-amylate de sodium ou le méthylate et l'éthylate de sodium.

Le choix particulier, parmi les bases appartenant à la classe définie ci-dessus, est déterminé par des considérations d'ordre économique, de sécurité et d'hygiène du travail. C'est ainsi que les alkoxydes sont préférés aux hydrures, le tert-butylate de potassium ou sodium étant employé de préférence.

On a pu déterminer que la proportion de la base utilisée doit être égale ou supérieure à la quantité stoechiométrique requise. En réalité, les rendements les meilleurs ont été obtenus en utilisant la base en excès.

Les temps de réaction observés sont relativement courts.

C'est ainsi qu'à des températures comprises entre environ 70° et 80°C, les temps de réaction peuvent être de l'ordre de 2 à 3 heures lorsque la base utilisée est l'hydrure de sodium dans un mélange de tétrahydrofuranne et d'hexaméthyl-triamidure de phosphore.

Bien entendu, la température exerce une influence déterminante sur les temps de réaction. La réaction, exothermique en soi, peut s'effectuer à une température de la température ambiante. Des températures comprises entre 20° et 90°C environ, sont employées de préférence. A des températures inférieures, les temps de réactions deviennent trop longs, tandis qu'à des températures dépassant la limite supérieure indiquée, il y a formation de produits secondaires indésirables.

Comme indiqué plus haut, la réaction s'effectue dans un solvant organique inerte. A cet effet, on peut utiliser des solvants organiques tels des éthers, comme le tétrahydrofuranne ou l'éther diisopropylique, des amides telles la diméthylformamide ou l'hexaméthyl-triamidure de phosphore. Des mélanges desdits solvants peuvent également être employés. Selon un mode d'exécution particulier, on utilise comme base le tert-butylate de potassium et en tant que solvant la diméthylformamide ou un mélange de diméthylformamide et tétrahydrofuranne.

L'invention est illustrée à l'aide des exemples suivants dans lesquels les températures sont indiquées en degrés centigrades.

**Exemples**

L'addition de l'alcool diallylique approprié à un mélange d'hydrure de potassium (1,1 équivalents), dans l'hexaméthyl-triamidure de phosphore (HMPA; 2 ml/mM d'alcool) à 20° sous atmosphère d'azote, fournit l'alkoxyde correspondant qui est ensuite chauffé directement dans le même solvant à 80° pendant 2 heures.

Le mélange de réaction est ensuite traité avec une solution aqueuse saturée de chlorure d'ammonium et extrait à l'éther.

Les extraits combinés sont soumis aux traitements usuels de lavage, neutralisation et évaporation des parties volatiles. Les cétones désirées sont enfin obtenues par distillation fractionnée du résidu formé. Le tableau ci-dessous résume les résultats obtenus.

| Produits de départ (alcools diallyliques) | Produits finaux (cétones insaturées) | Rendement (%) | Rapport pondéral*) |
|---|---|---|---|
| 1. 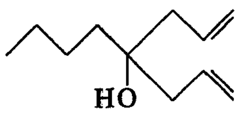 | 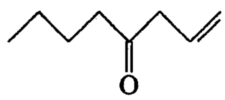 + 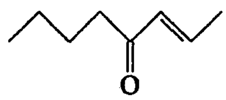 | 75 | 3:1 |
| 2. 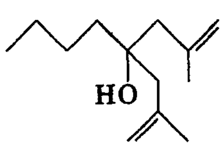 | 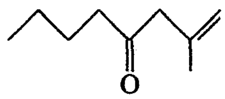 + 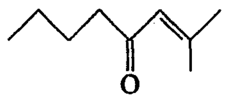 | 79 | 2:1 |
| 3. 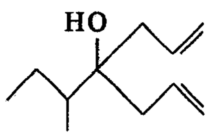 | 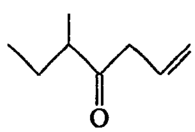 + 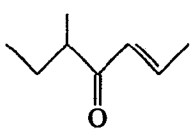 | 79 | 4:1 |
| 4. 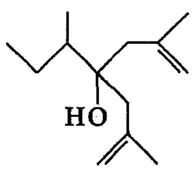 | 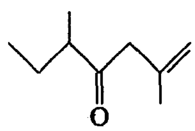 + 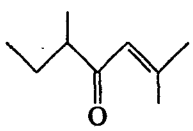 | 82 | 3:2 |
| 5. 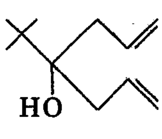 | 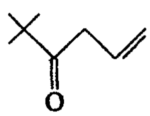 + 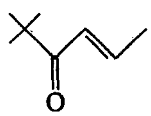 | 83 | 3:1 |
| 6. 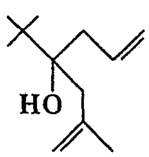 | 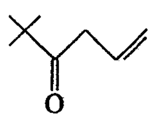 + 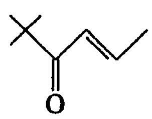 | 79 | 3:1 |
| 7. 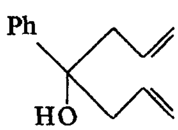 | 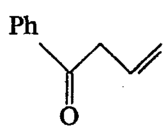 + 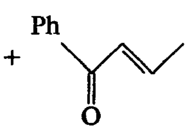 | 84 | 5:1 |
| 8. 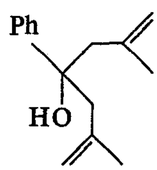 | 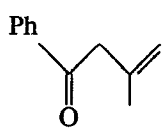 + 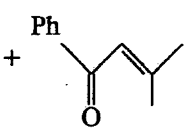 | 82 | 3:1 |

*) relatif à la présence, dans le mélange de cétones obtenues, de l'isomère *β,γ*-par rapport à l'isomère *α,β*-.

Ci-après sont indiqués les caractères analytiques des composés obtenus ainsi que ceux des produits de départ utilisés pour leur préparation.

1. 4-Butyl-1,6-heptadiène-4-ol
Eb. 83–85°/15 Torr;
IR: 3480, 3090, 1640, 1000, 920 et 880 $cm^{-1}$;
RMN: 0,90; 0,99; 1,32; 1,43 et 1,80 (9H); 1,55 (s, 1H); 2,23 (d, J = 7 Hz, 4H); 4,97 et 5,20 (m, 4H); 5,83 (m, 2H) $\delta$ ppm;
SM:m/e: 127(10), 111(3), 85(100), 69(30), 57(94).

1-Octène-4-one et (E)-2-Octène-4-one
Eb. 53–56°/15 Torr et Eb. 81°/15 Torr;
IR: 1700, 1680, 1640, 1370, 1300, 1200, 980 et 922 $cm^{-1}$;
RMN: 0,92; 1,85–2,50; 3,13; 4,92–5,25; 5,92; 6,10 et 6,80 $\delta$ ppm;
SM:$M^+$:126; m/e: 111(13), 85(15), 84(18), 69(100), 57(28), 41(56).

2. 4-Butyl-2,6-diméthyl-1,6-heptadiène-4-ol
Eb. 101–102°/15 Torr;
IR: 3560, 3080, 1640, 1380, 1130, 1070, 892 et 780 $cm^{-1}$;
RMN: 0,90; 1,01; 1,37; 1,43 et 1,82 (9H); 1,62 (s, 1H); 1,84 (6H, m); 2,23 (4H, s); 4,77 et 4,93 (4H, m) $\delta$ ppm;
SM:m/e: 141(9), 85(100), 69(3), 57(92), 55(24).

2-Méthyl-1-octène-4-one et 2-méthyl-2-octène-4-one
Eb. 60–64°/15 Torr;
IR: 3100, 1715, 1690, 1650, 1622, 1365, 1174, 1040 et 898 $cm^{-1}$;
RMN: 0,90; 1,75; 1,80–2,40; 3,09; 4,80; 4,90; 6,03 $\delta$ ppm;
SM:$M^+$ = 150(5); m/e: 125(1), 85(61), 83(45), 57(100), 41(39).

3. 4-(1-Méthylpropyl)-1,6-heptadiène-4-ol
Eb. 88–89°/15 Torr;
IR: 3500, 3080, 1640, 1380, 1000, 915 et 760 $cm^{-1}$;
RMN: 0,85–2,0 (9H); 1,53 (s, 1H); 2,24 (d, J = 7 Hz, 4H); 4,96 et 5,21 (m, 4H); 5,90 (m, 2H) $\delta$ ppm;
SM:m/e: 127(7), 111(4), 85(49), 69(39), 57(100).

5-Méthyl-1-heptène-4-one et (E)-5-méthyl-2-heptène-4-one
Eb. 52–55°/15 Torr;
IR: 3100, 1710, 1675, 1636, 1440, 1380, 1050, 996 et 920 $cm^{-1}$;
RMN: 0,70–2,80; 3,20; 5,00; 5,23; 5,62–6,30; 6,90 $\delta$ ppm;
SM:$M^+$ = 126(4); m/e: 111(2), 98(3), 85(14), 69(100), 57(41), 41(57).

4. 2,6-Diméthyl-4-(1-méthylpropyl)-1,6-heptadiène-4-ol
Eb. 102–105°/12 Torr;
IR: 3560, 3080, 1640, 1380, 1070, 1000, 892 et 760 $cm^{-1}$;
RMN: 0,85–2,0 (9H); 1,68 (s, 1H); 1,83 (m, 6H); 2,22 (m, 4H); 4,75 et 4,92 (m, 4H) $\delta$ ppm;
SM:m/e: 141(7), 85(56), 57(100), 55(25), 41(17).

2,5-Diméthyl-1-heptène-4-one et 2,5-diméthyl-2-heptène-4-one
Eb. 62–64°/15 Torr;
IR: 3090, 1710, 1690, 1624, 1460, 1380, 1040 et 898 $cm^{-1}$;
RMN: 0,72–2,80; 3,17; 4,80; 4,93 et 6,13 $\delta$ ppm;
SM:$M^+$ = 140(7); m/e: 85(75), 83(75), 57(100), 55(32) 41(32).

5. 4-(1,1-Diméthyléthyl)-1,6-heptadiène-4-ol
Eb. 80–83°/15 Torr;
IR: 3090, 3050, 1640, 1400, 1370, 1000, 918 et 860 $cm^{-1}$;
RMN: 0,97 (s, 9H); 1,57 (s, 1H); 2,37 (d, J = 7 Hz, 4H); 4,95 et 5,16 (m, 4H); 5,93 (m, 2H) $\delta$ ppm;
SM:m/e: 127(10), 111(5), 85(32), 69(71), 57(100).

2,2-Diméthyl-5-héxeène-3-one et (E)-2,2-diméthyl-4-héxène-3-one
Eb. 48–50°/15 Torr;
IR: 3100, 1700, 1635, 1480, 1400, 1370, 1320, 1300, 1120, 1060, 925 et 790 $cm^{-1}$;
RMN: 1,15 (s, 9H); 1,90 (d, J = 6 Hz); 3,27 (d, J = Hz); 5,00; 5,22; 5,62–6,25; 6,80 $\delta$ ppm;
SM:$M^+$ = 126(3); m/e: 98(1), 85(15), 69(70), 57(100), 41(72).

6. 2,6-Diméthyl-4-(1,1-diméthyléthyl)-1,6-heptadiène-4-ol
Eb. 99–101°/15 Torr;
IR: 3080, 3060, 1640, 1400, 1275, 1090, 995 et 892 $cm^{-1}$;
RMN: 0,98 (s, 9H); 1,77 (s, 1H); 1,87 (m, 6H); 2,30 (m, 4H); 4,79 et 4,88 (m, 4H) $\delta$ ppm;
SM:m/e: 141(5), 97(1), 85(23), 69(1), 57(100), 55(36), 41(20).

2,2,5-Triméthyl-5-hexène-3-one et 2,2,5-triméthyl-4-héxène-3-one
Eb. 60–64°/15 Torr;
IR: 3090, 1710, 1686, 1652, 1622, 1480, 1370, 1320, 1070, 1008 et 893 $cm^{-1}$;
RMN: 1,17 (s, 9H); 1,77; 1,90; 2,10; 3,23; 4,75; 4,92; 6,30 $\delta$ ppm;
SM:$M^+$ = 140(2); m/e: 85(17), 83(36), 57(100), 55(22), 41(31).

7. 4-Phényl-1,6-heptadiène-4-ol
Eb. 127–130°/15 Torr;
IR: 3500, 3080, 2990, 1640, 1500, 1448, 1000, 920 et 704 $cm^{-1}$;
RMN: 2,24 (s, 1H); 2,58 (m, 4H); 4,85–6,0 (6H); 7,35(m, 5H) $\delta$ ppm;
SM:m/e: 147(18), 105(100), 91(1,3), 77(30), 51(6), 41(9).

1-Phényl-3-butène-1-one et (E)-1-phényl-2-butène-1-one
Eb. 105–110°/15 Torr;
IR: 1680, 1640, 1600, 1580, 1450, 1338, 1210, 1180, 1004, 920, 760 et 697 $cm^{-1}$;
RMN: 2,00 (d, J = 6 Hz); 3,72 (d, J = 7 Hz); 5,10; 5,30; 5,80–6,50; 7,30–8,10 $\delta$ ppm;
SM:$M^+$ = 146(58); m/e: 131(27), 117(7), 105(100), 77(78), 69(57), 51(38).

8. 2,6-Diméthyl-4-phényl-1,6-heptadiène-4-ol
Eb. 73–77°/0,01 Torr;
IR: 3550, 3080, 3040, 1640, 1498, 1443, 1380, 1070, 1030, 900, 730 et 700 $cm^{-1}$;
RMN: 1,40 (m, 6H); 2,53 (s, 1H); 2,60 (s, 4H); 4,68 et 4,86 (m, 4H) $\delta$ ppm;
SM:m/e: 161(8), 106(6), 105(100), 77(24), 55(3).

3-Méthyl-1-phényl-3-butène-1-one et 3-méthyl-1-phényl-2-butène-1-one
Eb. 115–120°/15 Torr;
IR: 3080, 2995, 1700, 1675, 1620, 1600, 1580, 1450, 1380, 1250, 1210, 1180, 1010, 900, 760, 695 $cm^{-1}$;
RMN: 1,83; 2,03; 2,23; 3,70; 4,85; 4,98; 6,73; 7,25–8,10 $\delta$ ppm;
SM:$M^+$ = 160(6); m/e: 145(3), 105(100), 77(47), 5(16), 39(4).

Suivant le procédé de l'invention, les cétones insaturées obtenues se présentent sous forme de mélanges d'isomères $\alpha,\beta$- et $\beta,\gamma$-insaturés. Ces isomères peuvent être séparés par chromatographie en phase gazeuse ou alternativement peuvent être traités au moyen d'un agent d'isomérisation acide usuel pour fournir l'isomère $\alpha,\beta$. Comme agent d'isomérisation, il convient tout particulièrement de mentionner l'acide p-toluènesulfonique ou une terre de diatomées acide.

Les 1,6-heptadiène-4-ols, utilisés comme produits de départ dans le procédé de l'invention, peuvent être préparés avec des bons rendements par traitement de l'ester carboxylique approprié avec le chlorure d'allyle ou de méthallyle dans les conditions d'une réaction de type de Grignard dans le tétrahydrofuranne.

**Revendications**

1. Procédé pour la préparation de cétones $\alpha,\beta$- et $\beta,\gamma$-insaturées de formule

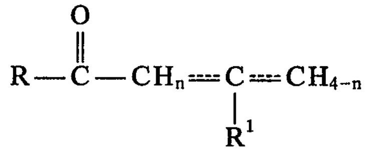

(II)

possédant une double liaison dans l'une des positions indiquées par les pointillés et dans laquelle le symbole R sert à définir un radical alkyle primaire, secondaire ou tertiaire ou un reste phényle, substitué ou non, le symbole $R^1$ représente un atome d'hydrogène ou un radical alkyle et l'indice n vaut 1 ou 2, caractérisé en ce qu'on traite au moyen d'une base forte, et dans un solvant organique inerte, un carbinol diallylique de formule

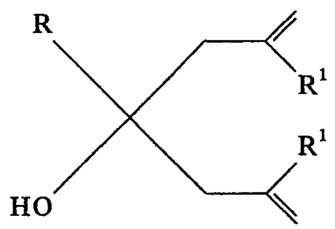

(I)

dans laquelle les symboles R et $R^1$ ont le sens indiqué ci-dessus.

2. Procédé suivant la revendication 1, caractérisé en ce que la base forte est un hydrure ou un alkoxyde.

3. Procédé suivant la revendication 2, caractérisé en ce qu'on utilise comme alkoxyde le tert-butylate de sodium ou potassium.

4. Procédé suivant l'une quelconque des revendications précédentes, caractérisé en ce que la réaction est effectuée au moyen de tert-butylate de potassium dans l'hexaméthyltriamidure de phosphore et à une température comprise entre 50° et 90°C.

## Patentansprüche

1. Verfahren zur Herstellung von $\alpha,\beta$- und $\beta,\gamma$-ungesättigten Ketonen der Formel

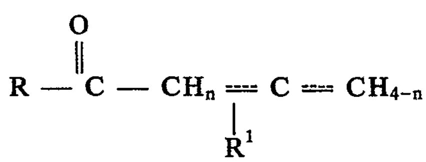

(II)

die in einer der durch die gestrichelten Linien angegebenen Stellungen eine Doppelbindung hat, und worin R einen primären, sekundären oder tertiären Alkylrest oder eine substituierte oder unsubstituierte Phenylgruppe, $R^1$ ein Wasserstoffatom oder ein Alkylrest, bedeutet, und Index n für 1 oder 2 steht, dadurch gekennzeichnet ist, daß ein Diallyl-Carbinol der Formel

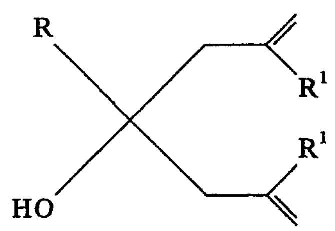

(I)

worin R und $R^1$ die oben angegebene Bedeutung haben, mit einer starken Base in einem inerten organischen Lösungsmittel, behandelt wird.

2. Verfahren gemäß Anspruch 1, dadurch gekennzeichnet, daß die starke Base ein Hydrid oder ein Alkoxyd ist.

3. Verfahren gemäß Anspruch 2, dadurch gekennzeichnet, daß das Alkoxyd Natrium oder Kalium tert-Butoxyd ist.

4. Verfahren gemäß einem der vorangehenden Ansprüche, dadurch gekennzeichnet, daß die Reaktion mittels Kalium tert-Butoxyd in Phosphor-Hexamethyltriamide und bei einer Temperatur von zwischen 50 und 90°C durchgeführt wird.

## Claims

1. Process for the preparation of $\alpha,\beta$- and $\beta,\gamma$-unsaturated ketones of formula

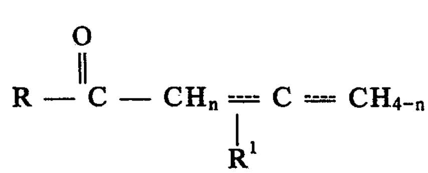

(II)

possessing a double bond in one of the positions indicated by the dotted lines and wherein symbol R

designates a primary, secondary or tertiary alkyl radical or a substituted or unsubstituted phenyl group, symbol $R^1$ represents a hydrogen atom or an alkyl radical and index n stands for 1 or 2, characterized in that a diallyl carbinol of formula

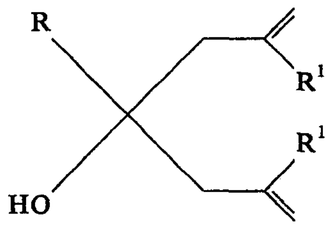

(I)

wherein symbols R and $R^1$ have the above given meaning, is treated with a strong base in an inert organic solvent.

2. Process according to claim 1, characterized in that the strong base is a hydride or an alkoxide.

3. Process according to claim 2, characterized in that the alkoxide is sodium or potassium tert-butoxide.

4. Process according to any of the preceding claims, characterized in that the reaction is effected by means of potassium tert-butoxide in phosphorous hexamethyltriamide and at a temperature of between 50 and 90°C.